# EUROPEAN PATENT SPECIFICATION

(11) **EP 0 866 654 B1**
(45) Date of publication and mention of the grant of the patent: **14.11.2007**
(21) Application number: 96936025.4
(22) Date of filing: 27.09.1996
(51) Int. Cl.: A01K 67/027, A61K 48/00, C12N 5/10, C12N 7/00, C12N 15/51, C12Q 1/68, C07K 14/02, A61K 38/21, C07K 14/565, A61P 1/00

(54) **METHOD FOR TREATING HEPATITIS VIRUS INFECTION**
METHODE FÜR DIE BEHANDLUNG EINER HEPATITISVIRUSINFEKTION
METHODE DE TRAITEMENT DE L'INFECTION PAR LE VIRUS DE L'HEPATITE

(30) Priority: 27.09.1995 US 4357 P
(43) Date of publication of application: 30.09.1998
(73) Proprietor: THE GENERAL HOSPITAL CORPORATION, Boston, MA 02114 (US)
(72) Inventor: TAKAHASHI, Hiroshi, Winchester, MA 01890 (US)
(74) Representative: Dzieglewska, Hanna Eva
(86) International application number: PCT/US1996/015554
(87) International publication number: WO 1997/011598

(56) References cited:
- WO-A-94/27556
- US-A- 5 346 696
- US-A- 5 378 605
- TAKAHASHI H, FUJIMOTO J, HANADA S, ISSELBACHER KJ.: "Acute hepatitis in rats expressing human hepatitis B virus transgenes." PROC NATL ACAD SCI U S A. 1995 FEB 28;92(5):1470-4., XP002190181
- KASAMA K. ET AL.: "Pharmacokinetics and biologic activities of human native and asialointerferon-beta s." J INTERFERON CYTOKINE RES, vol. 15, no. 5, May 1995 (1995-05), pages 407-415, XP008004854
- BOSE S, HICKMAN J.: "Role of the carbohydrate moiety in determining the survival of interferon in the circulation." J BIOL CHEM. 1977 DEC 10;252(23):8336-7., XP008004757
- DRAGSTEN P R ET AL: "DRUG DELIVERY USING VESICLES TARGETED TO THE HEPATIC ASIALOGLYCOPROTEIN RECEPTOR" BBA - GENERAL SUBJECTS, ELSEVIER SCIENCE PUBLISHERS, NL, vol. 926, 1987, pages 270-279, XP002931318 ISSN: 0304-4165
- PROC. NATL. ACAD. SCI. U.S.A., September 1989, Vol. 86, BEHR et al., "Efficient Gene Transfer Into Mammalian Primary Endocrine Cells with Lipopolyamine-Coated DNA", pages 6982-6986.
- JOURNAL OF HEPATOLOGY, Vol. 23, No. 1, 1995 (Munksgaard, Denmark), KIM et al., "Hepatitis G Virus (HGV), a New Hepatitis Virus Associated with Human Hepatitis", POSTGRADUATE COURSE AND ABSTRACT OF THE 30TH ANNUAL MEETING OF THE EUROPEAN ASSOCIATION FOR THE STUDY OF THE LIVER, 20-23 August, Copenhagen, Denmark.

## Description

### Background of the Invention

Hepatitis B virus (HBV) infection is a worldwide health problem. It causes a wide spectrum of pathologies ranging from inapparent infection to fatal hepatocellular diseases (Tiollais et al., Nature 317:489, 1985). The HBV virion is composed of an envelope, which carries the hepatitis B surface antigen (HBsAg), and a nucleocapsid. The nucleocapsid encloses a circular, partially double-stranded 3.2 kb DNA, which replicate via a RNA intermediate. The nucleocapsid is formed by the hepatitis B core antigen. When virions are present in the blood, an addition soluble antigen related to the capsid, the hepatitis B e antigen (HBeAg), is generally detected in the serum. Several studies have suggested that HBV is not directly hepatocytopathic and that host immune response to viral antigens presented on the surface of infected liver cells may play an important role in pathogenesis (Mondelli et al., Immunol. 129:2773, 1982; Mondelli et al., Arch. Pathol. Lab. Med. 112:489, 1988; Chisari et al., Microb. Pathog. 6:311, 1989).

The lack of suitable animal models for hepatitis B have hindered understanding of the molecular mechanisms responsible for hepatocyte death and viral clearance (Ochiya et al., Proc. Nat'l. Acad. Sci. USA 86:1875, 1989; Gripon et al., J. Virol. 62:4136, 1988). Germ-line transgenic mouse models have been produced to investigate the pathogenesis of HBV infection, but these lives are immunologically tolerant to HBV antigens and do not spontaneously develop hepatitis (Moriyama et al., Science 248:361, 1990). Hepatitis must be induced in these animals by complicated, multi-step process involving, e.g., the priming lymphocytes with HBV proteins in syngenic animals and adoptive transfer of the primed cells in vivo (Moriyama et al., supra Ando et al., J. Exp. Med. 178:1541, 1993).

### Summary of the Invention

The invention features a method for treating an infection of cells bearing the asialoglycoprotein receptor with a modified form of naturally occurring interferon or interferon produced in a mammalian cell or eukaryotic cell. The method entails administering an effective amount of asialo interferon. This asialo interferon has been prepared by converting an interferon produced in a eukaryotic cell to the asialo form by treatment with neuramidase or by other methods known to those skilled in the art. Thus, the method entails treatment with an interferon produced in a eukaryotic cell, which interferon has been modified by removing a terminal sialic residue. In various preferred embodiments, the interferon is interferon β and the interferon is interferon α, the interferon used to produce asialo interferon was produced by a human fibroblast, and the interferon used to produce the asialo interferon was produced recombinantly.

As described in the experimental results presented further below, this aspect of the invention may be investigated by using an animal hepatitis model that exhibits a sequence of pathological changes similar to those observed in humans suffering from HBV associated acute hepatitis. This may be a non-human mammal having hepatocytes transfected in vivo with replicating human hepatitis viral DNA, which mammal exhibits one or more symptoms of human hepatitis. The mammal may be a rodent, e.g. a mouse or a rat. The hepatitis virus may be hepatitis B, hepatitis C, and hepatitis G. A method for creating a somatic transgenic mouse having hepatocytes transfected with replicating human hepatitis viral DNA entails contacting hepatocytes with a DNA molecule, which molecule includes human hepatitis viral DNA, complexed with a catonic lipid, whereby the complex binds to hepatocytes so that the DNA enters the cells.

Such an animal model may also be used for screening candidate drugs for ability to treat hepatitis. Such a screening method entails obtaining a non-human mammal having hepatocytes transfected in vivo with replicating human hepatitis viral DNA, which mammal exhibits one or more symptoms of human hepatitis (e.g., viremia and viral protein expression, antibody production, elevation of liver-derived enzymes such as GDT, GPT, LDH, y-GTP in serum; histological changes including necrosis or apoptosis of hepatocytes and lyphocyte infiltration; and replication of virus in the liver) and administering to the animal either a candidate drug or a placebo (or a second candidate drug or a known effective drug) and then measuring a symptom of hepatitis or the level of infection (as indicated by the presence of virion or a viral protein or a viral nucleic acid) in the mammal and determining whether said candidate drug diminishes a symptom of hepatitis or the level of infection compared to a mammal treated with placebo (or a second candidate drug or a known effective drug).

### Brief Description of the Drawings

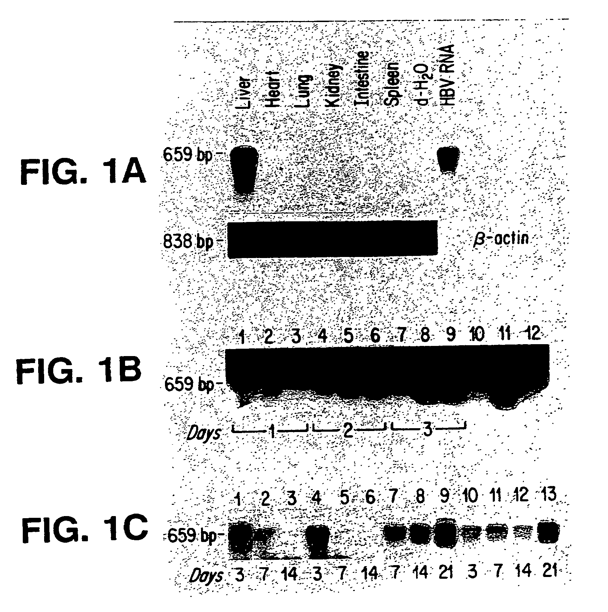

Figure 1A is a photograph of the results of Southern analysis of the expression of HBV genes in transfected rats. RNA was isolated from the indicated tissues and a 659-bp HBV-specific sequence was amplified by RT-PCR and detected by Southern analysis. Total RNA isolated from a pHBV-HTD transfected Huh-7 hepatoma cell line served as a positive control for HBV RNA. Amplification of an 838-bp β-actin fragment served as a control for sample loading an integrity.
Figure 1B is a photograph of the results of Southern analysis used to measure HBV virion in rat sera. Sera were obtained from three rats at 1 day (lanes 1-3), 2 days (lanes 4-6), and 3 days (lanes 7-9) after transfection with pHBV-HTD. Untransfected normal rat serum (lane 10) and pHBV-HTD DNA (100 ng; lane 12) were used as negative controls, and the culture supernatant from a pHBV-HTD transfected Huh-7 hepatoma cell lime served as a positive control. This PCR assay was specific for the HBV viral particle because DNA from the complete viral particle was detected (lane 11), but pHBV-HTD plasmid DNA was not detectable under the same conditions (lane 12).
Figure 1C is a photograph of the results of PCR Southern analysis used to measure HBV virion in sera of normal (lanes 1-6) and athymic nude rats (lanes 7-13). Sera were obtained from rats at 3 days (lanes 1, 4, and 10), 7 days (lanes 2, 5, 7, and 11), 14 days (lanes 3, 6, 8, and 12), and 21 days (lanes 9 and 13) after the pHBV-HTD transfection, and HBV virion DNA was detected as described above.

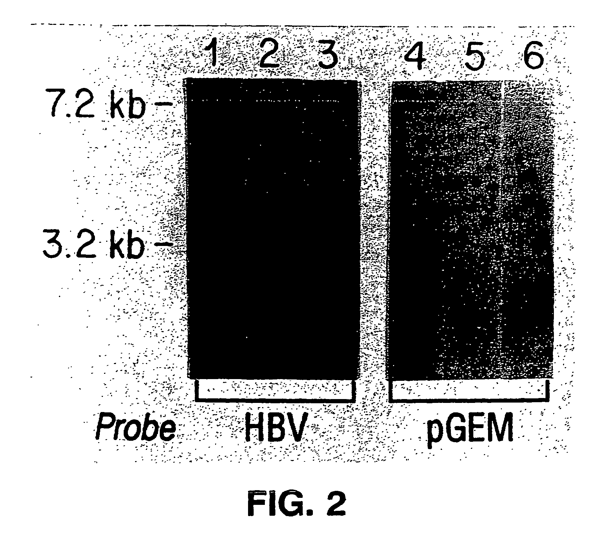

Figure 2 is a photograph of the results of Southern blot analysis of HBV DNA in rat liver. DNA was extracted from the liver 1 day (lanes 1 and 4), 2 days (lanes 2 and 5), or 3 days (lanes 3 and 6) after the liver was transfected with the adwR9 head-to-tail heterodimer of an HBV construct. Southern blots containing liver DNAs digested with *Eco*RV were hybridized first with an HBV-specific probe (lanes 1-3), stripped, and then rehybridized with a vector-specific probe (lanes 4-6).

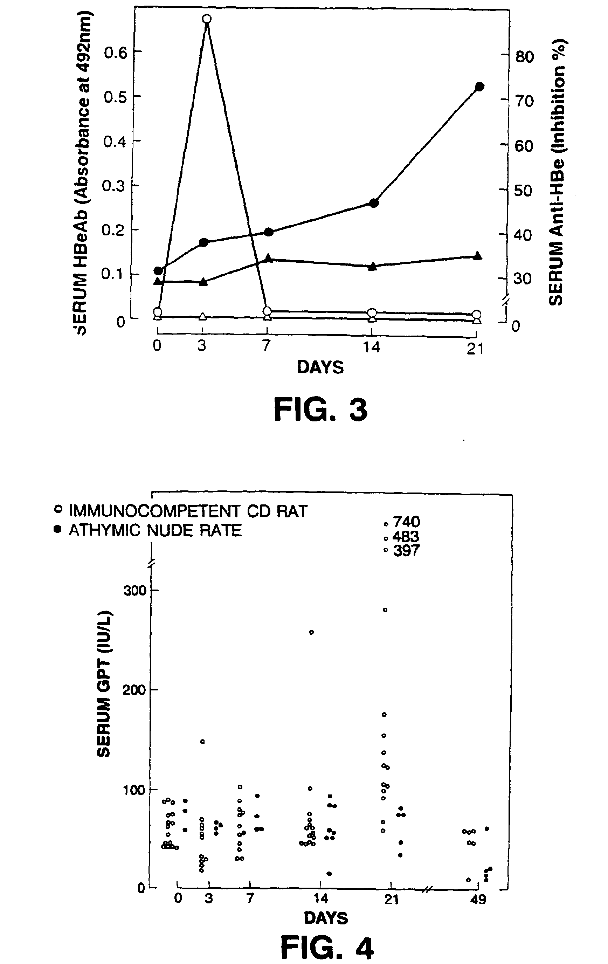

Figure 3 is a graph depicting the results of an analysis of serum HBeAg level (open symbols) and anti-HBe antibody titer (closed symbols) in rats transfected with pHBV-HTD (circles) or pGEM-7Zf(+) (triangles). The relative concentrations of HBeAg and anti-HBe are expressed by *A*₄₉₂ and percent inhibition, respectively, as described herein. Specimens whose *A*₄₉₂ value is equal to or greater than the cutoff value of 0.065 (mean of the negative control plus the factor 0.06) are considered to be positive for HBeAg, and those with a percent inhibition value equal to or greater than 50% are considered to be positive for anti-HBe.
Figure 4 is a graph illustrating serum GPT levels in normal CD rats (○) and in athymic nude rats (●) transfected with pHBV-HTD.

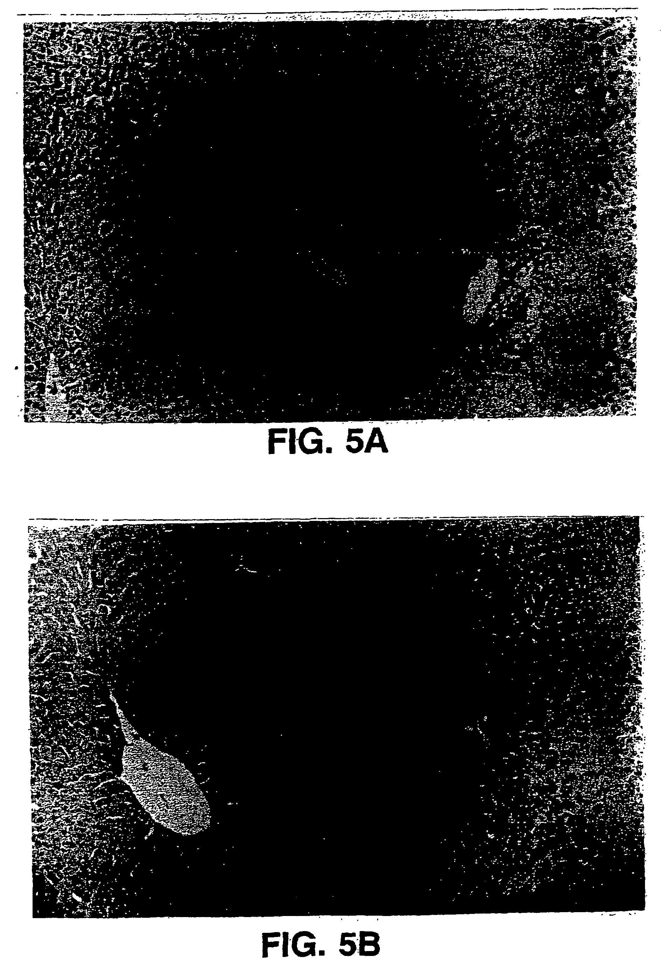

Figure 5A is a photograph of the results of hematoxylin and eosin staining of a paraffin section of rat liver transfected in vivo with pHBV-HTD (x80).
Figure 5B is a photograph of the results of hematoxylin and eosin staining of a paraffin section of rat liver transfected with pGEM-7Zf(+) (x80). No hepatocellular injury was observed.

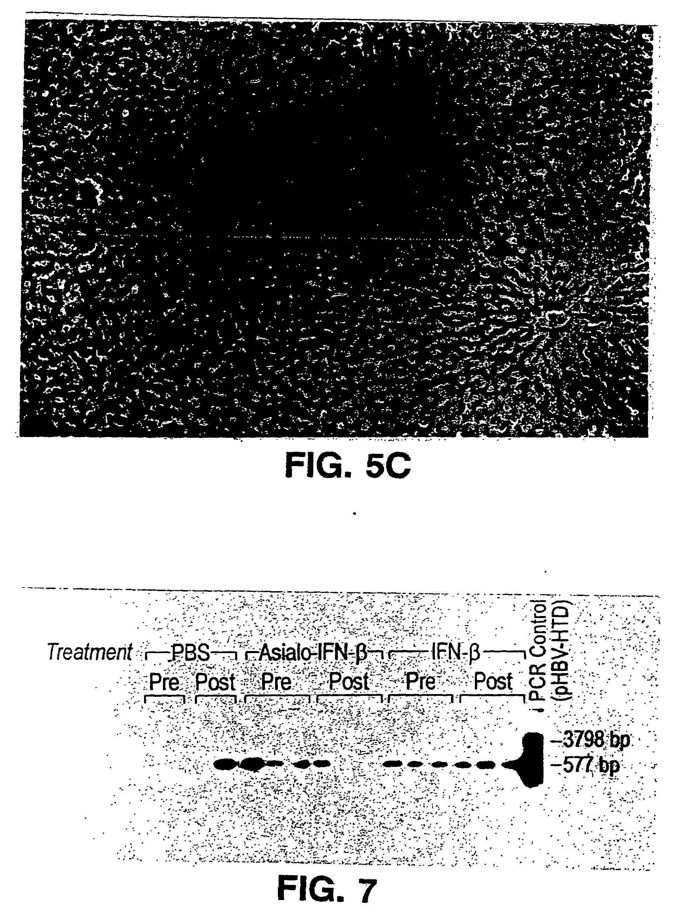

Figure 5C is a photograph of the results of hematoxylin and eosin straining of a paraffin section of liver from a nude rat transfected in vivo with pHBV-HTD (x80).

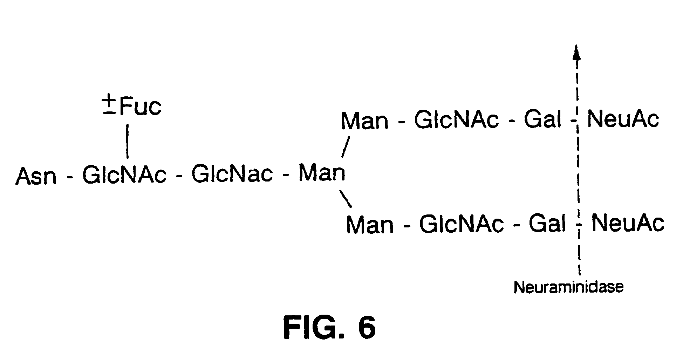

Figure 6 is a schematic illustration of the structure of natural human IFN-β. Also illustrated are the cleavage sites of typical biantennary complex-type sugar chains of natural human IFN-β by neuraminidase. Abbreviations: Fuc, fucose; GlcNAc, N-acetylglucosamine; Man, mannose; Gal, galactose; NeuAc, N-acetylneuraminic acid.
Figure 7 is a photograph illustrating the results of Southern blot analysis used to measure the in *vivo* effect of asialo IFN-β in nude mice model of human HBV. "Pre" indicates serum HBV detected by PCR-Southern analysis 7 days after in vivo transfection just before the treatment. Note that asialo IFN-β demonstrated a substantial anti-viral effect. "Post" indicates serum HBV detected by PCR-Southern analysis 14 days after in vivo transfection and at the end of 7 days treatment. "PBS" indicates phosphate buffered saline was administered. "Asialo IFN-β" indicates that natural sialylated IFN-β was administered.

### Detailed Description of the Invention

Described below are four sets of experiments. The first set of experiments is related to the production of a rat model of acute hepatitis. The second set of experiments concerns a mouse model of acute hepatitis. The third set of experiments concerns a method for treating hepatitis with asialo-interferon β. The fourth set of experiments concerns the effect of asialo-interferon B on HBV transfected human hepatoma cells.

### Preparation of the Rat Model

A head-to-tail homodimer of cloned HBV (pHBV-HTD), adw sub-type, was constructed and inserted into the EcoRI site of the pGEM-72f(+) vector (Promega, Madison WI) as described by Blum et al., J. Virol. 65:1836, 1991 and Blum et al., Hepatology 14:56, 1991. (The cloning of the adw subtype is described in Valenzuela et al., The nucleotide sequence of the hepatitis B viral genome and the identification of the major viral genes, in Animals Virus Genetics, Fields et al., eds., Academic Press, New York, 1980, p.57.) In some cases, a head-to-tail heterodimer of HBV, adwR9, a replication-competent HBV construct similar to pHBV-HTD was used for transfection (Blum et al., J. Virol. 65:1836, 1991 and Blum et al., Hepatology 14:56, 1991).

A two-thirds hepatectomy was performed according to the Higgins-Anderson method (Higgins et al., Arch. Pathol. 12:186, 1931) 24 h before in vivo transfection, since hepatectomy increases the expression of the HBV transgene. The cloned HBV constructs were directly delivered into rat livers in vivo by using a membrane fusion-promoting cationic lipid, dioctadecylamidoglycylspermine (Behr et al., Proc. Nat'l. Acad. Sci. USA 86:6982, 1989). Fifty micrograms of pHBV-HTD or pGEM-7Zf(+) vector was mixed with 250 µg of the cationic lipid in 500 µl of sterile saline (0.154 M NaCl) and allowed to form a DNA-cationic lipid complex. Animals were anesthetized with ether and injected with the DNA-cationic lipid complex into the right median lobe of the liver while their portal veins were temporarily ligated by hemostatic forceps.

In addition to the above-described method, 20 µG of pHBV-HTD (or another selected DNA molecule) can be complexed with 50 µg asialofetuin-poly-L-lysine and 100 µg cationic liposome in 250 µg HBSS and injected as described above.

### HBV RNA in Rat Tissues.

To investigate the expression of HBV in transfected rats, total RNA was extracted from selected tissues of rats sacrificed 3 days after in vivo transfection with pHBV-HTD and amplified by RT-PCR (Chelly et al., Nature 333:859, 1988). As shown in Figure 1A, a 659-bp PCR fragment of HBV transcript was detected in the liver but not in other tissues. In this experiment total RNA isolated from a pHBV-HTD transfected Hub-7 hepatoma cell line (lane labelled "HBV RNA") served as a positive control.

The level of HBV expression was measured as follows. Total RNA was extracted from the liver, heart, lung, kidney, intestine, and spleen from rats transfected with pHBV-HTD and digested with RNase-free DNase I (2 units/µg of RNA) (Promega) for 30 min. at 37°C. cDNAs were synthesized by extension of antisense primers with reverse transcriptase (BRL) in a mixture containing 2 µg of total RNA. PCR of the cDNA was performed in a final volume of 100 µl containing 2.5 mM NgCl₂ and 100 pmol of each primer. The amplification cycles were 95°C for 30 s, 50°C for 1 min, and 72°C for 1 min. After 35 cycles, the PCR products were separated by electrophoresis on a 1.5% agarose gel and transferred to Hybond-N+ nylon membranes (Amersham). The Southern blot was hybridized with a ³²P-labeled HBV-specific restriction fragment (Aat II fragment of pHBV-HTD; 3221 bp). The primers used for amplification were located within the S open reading frame of HBV (sense primer, 5'-TGCGGGTCACCATATTCTTGGGAACAAGA-3' (SEQ ID NO:1); antisense primer, 5'-AGTCTAGACTCTGCGGTATTGTGAGGATTCTTG-3' (SEQ ID NO:2), which yielded a 659-bp fragment. β-Actin primers that amplified an 838-bp fragment were used as a control (sense primer, 5'-ATCTGGCACCACACCTTCTACAATGAGCTGCG-3'; SEQ ID NO:3); antisense primer, 5'-CGTCATACTCCTGCTTGCTGATC-CACATCTGC-3'; SEQ ID NO:4). Total RNA obtained from a human hepatocellular carcinoma cell line (Huh-7) transfected with pHBV-HTD *in vitro* (Blum et al., Hepatology 14:56, 1991) served as a positive control for detection of HBV RNA.

### HBV Virion in Rat Sera

The presence of HBV virions in serum was assessed by detecting HBV DNA by PCR after DNaseI treatment of rat sera and immunoprecipitation of HBV virions with anti-HBsAg-conjugated beads. Sera were positive for virions in 18 to 21 rats. The amount of HBV detected in the serum increased during the first 3 days after transfection as assayed by increases in PCR amplifiable material detected by Southern analysis (Figure 1B, lanes 1-9) and then rapidly decreased and could no longer be detected 14 days after the transfection (Figure 1C, lanes 1-6).

HBV virion sera was measured as follows. Sera were first treated with 20 units of DNase I (Boehringer Mannheim) per ml at 37°C for 30 min to digest naked DNAs, such as pHBV-HTD plasmid. The HBV particles were then immunoprecipitated from the sera by using mouse anti-HBsAg antibody (5D3 monoclonal antibody) (Takahashi et al., J. Immunol. Methods 112:91, 1988) conjugated to azlactone-acrylamide copolymer beads (Pierce). HBV is immunoprecipitated by these antibody-conjugated beads because the complete HBV virion contains double-stranded 3.2-kb HBV DNA and carries HBsAg on its envelope. After extensive washing, HBV DNA was released from the beads by heating at 95°C for 5 min, amplified by PCR by using the same primers described above, and hybridized to the HBV probe in a Southern analysis to confirm the specificity of the PCR products (Liang et al., J. Clin. Invest. 84:1367, 1989).

As an alternative, PCR analysis can be performed as follows. PCR analysis of HBV is performed in a final volume of 50 µl with 2.5 mM MgCl₂ and 1 µM of each primer. The cycles are 95°C for 30 s, 50°C for 1 min and 72°C for 1 min. After 30 cycles, the PCR products are separated by electrophoresis on 1.5% agarose gel and transferred to Hybond-N⁺ nylon membranes (Amersham). The blot is hybridized with a ³²P-labeled HBV-specific restriction fragment (AatII fragment of pHBV-HTD; 3221bp) for Southern analysis. The following primers, located within the core open reading frame of HBV gene, are used for the detection of HBV: sense primer, GAGAATTCAAGCCTCCAAGCTGTGCCTGG (SEQ ID NO:5); anti-sense primer, GAAAGCTTCTGCGACGCGGCGATTGAGA (SEQ ID NO:6). These primers yield a 578 bp fragment. The following beta-actin primers are used as a control: sense primer, ATCTGGCACCACACCTTCTACAATGAGCTGCG (SEQ ID NO:7); anti-sense primer, CGTCATACTCCTGCTTGCTGATCCACATCTGC (SEQ ID NO:8). These primers yield a 1045 bp fragment.

### HBV Liver DNA

HBV DNA was detected by genomic Southern analysis of liver DNA isolated from rats transfected with adwR9. DNA bands of 7.2 kb and 3.2 kb were detected in *Eco*RV-digested genomic DNAs by hybridization with an HBV-specific probe (*Aat* II fragment of pHBV-HTD) (Figure 2, lanes 1-3). The 7.2 kb band was also seen in the same liver DNA blots that were rehybridized with a vector-specific probe [pGEM-7Zf(+) DNA digested by BamHI], but the 3.2 kb band was not detectable (lanes 4, 5, and 6). Since both the HBV genome (adw subtype; 3.2 kb) and the adwR9 HBV constructs (7.2 kb) have a single *Eco*RV site (Blum et al., Hepatology 14:56, 1991), the observed 3.2 kb band was not derived from the digested adwR9. These data confirm that HBV DNA was produced and present in an unintegrated form in the liver. In addition, the HBV DNA was detected at a similar intensity on days 1, 2, and 3 (lanes 1-3, respectively), although the adwR9 plasmid DNA in the liver rapidly decreased between day 1 and day 3. Thus, the presence of 3.2 kb HBV DNA after the clearance of the adwR9 construct from the liver indicates that HBV production may have been mediated by its own replication.

Genomic Southern analysis was performed as follows. Rat livers transfected with a head-to-tail heterodimer of HBV, adwR9, were used for analysis. DNA was extracted from the livers, digested with *Eco*RV, which cuts the HBV genome and adwR9 at a single site, and separated by electrophoresis through a 1% agarose gel. The DNA fragments were transferred to a Southern hybridization filter and the blots were first hybridized with an HBV-specific restriction fragment (*Aat* II fragment of pHBV-HTD), stripped, and then rehybridized with a vector-specific restriction fragment [pGEM-7Zf(+) DNA digested with *Bam*HI] for Southern analysis.

### Serum HBeAg and Anti-HBe Antibody Response in Transfected Rats

A representative time course of serum HBeAg level and anti-HBe antibody response in pHBV-HTD-transfected rats is shown in Figure 3. HBeAg was found in rat serum 3-7 days after liver transfection with pHBV-HTD and was followed by an increase in anti-HBe antibody titer by day 21. Neither HBeAg nor anti-HBe was found in the sera of mock-transfected rats transfected with pGEM-7Zf(+) (Figure 3).

HBeAg and anti-HBe antibody were measured as follows. Sera were collected from tail veins of rats. The presence of HBeAg and anti-HBe antibody were determined by "sandwich" and "competitive-binding" techniques, respectively, using commercially available ELISA kits (Abbott). The relative concentration of HBeAg was represented by the absorbance value of specimens at 492 nm (A₄₉₂). The level of anti-HBe was expressed as percent inhibition calculated by using the following formula; percent inhibition = [(mean A₄₉₂ of negative controls - A₄₉₂ of sample)/(mean A₄₉₂ of negative controls - A₄₉₂ of positive controls)] X 100.

### Serum Glutamic-Pyruvic Transaminase (GPT) Levels.

GPT activity in the serum was measured as an indicator of liver disease, since GPT is found primarily in the liver and released from the damaged hepatocytes. Serum GPT values were elevated in the majority of rats 2-3 weeks after HBV transfection [60 international units (IU)/l ± 5IU/l at day 0 and 210 IU/l ± 49 IU/l at day 21; mean ± SEM, *n* = 15] (Figure 4). No serum GPT elevation was observed in the mock-transfected rats (37 IU/l ± 18 IU/l at day 0 and 30 IU/l ± 12 IU/l at day 21, *n* = 3).

### Liver Histology.

Liver tissues were obtained from randomly chosen rats sacrificed 3-21 days after *in vivo* transfection with the pHBV-HTD, adwR9, or pGEM-7Zf(+) construct. Figure 5A demonstrates the liver tissue histology of a rat 21 days after *in vivo* transfection with the pHBV-HTD construct with a serum GPT level of 483 IU/l (see Figure 4). The parenchymal cells in the vicinity of the portal vein had disappeared and were replaced by the infiltration of lymphocytes. Other animals transfected with pHBV-HTD or adwR9 demonstrated similar histological changes. No hepatocyte death or lymphocyte infiltration was observed in the livers of mock-transfected rats (Figure 5B).

### In vivo Transfection of Athymic Nude Rats with pHBV-HTD.

To see if T lymphocytes are important for the induction of liver cell injury in the experimental animal model described herein, T-lymphocyte-deficient athymic nude rats were transfected with pHBV-HTD as described above. No serum GPT elevation was observed in any of these transfected animals (Figure 4) and their livers were histologically normal (Figure 5C). It is interesting to note that the serum level of HBV virions increased between 7 and 21 days in these nude rats (Figure 1C, lanes 7-13). This finding was in contrast to the serum HBV virion levels of normal rats, which rapidly decreased by 7-14 days after the transfection (Figure 1C, lanes 1-6).

### Characterization of the Model

After in vivo transfection of clone HBV DNA according to the above described technique, HBV RNA as well as 3.2-kb HBV DNA were present in the liver, and HBV virions were detected in the blood. Most importantly, HBeAg, a serological marker of active viral replication (Brechot et al., Lancet, ii:765, 1981; Hadziyannis et al., Hepatology 3:656, 1993), was found in rat sera, and its appearance was followed by an anti-HBe antibody response. These data indicate that HBV virions were actively produced and that an immune response to HBV gene products was elicited in rats transfected with the HBV constructs. Furthermore, the liver histology in these animals demonstrated severe hepatocellular injury characterized by hepatocyte death and lymphocyte infiltration when serum GPT values were elevated. Thus, HBV-induced pathogenesis in these transfected rats was characterized by the expression of HBV genes, the production of HBV virions, the increase of serum transaminase, and the characteristic histological findings. These pathological changes in rats transfected with HBV DNA are similar to those found in acute viral hepatitis induced by HBV in humans.

These studies used the head-to-tail dimer constructs of HBV (pHBV-HTD) and adwR9. These and other head-to-tail dimer constructs contain the HBV genome and endogenous viral enhancer/promoter elements that are sufficient for the production of complete virions in human hepatoma cell lines (Blum et al., J. Virol. 65:1836, 1991; Blum et al., Proc Nat'l Acad. Sci. USA 84:1005, 1987; Sureau et al., Cell 47:37, 1986; Yaginuma et al., Proc. Natl Acad. Sci. USA 84:2678, 1987; Yasinuma et al., Proc. Nat'l Acad. Sci. USA 84:2678, 1989) and the *in vitro* replication of HBV has been previously demonstrated in rat liver-derived cells (Shih et al., Proc. Natl. Acad. Sci USA 86:6323, 1989; Diot et al., J. Med. Virol. 36:93, 1992). However, there had been no previous studies to determine if the HBV gene would be expressed in rat liver *in vivo* after the direct transfection of these replication-competent constructs. The present data demonstrate the expression of HBV genes, the production of HBV particles, and the development of spontaneous hepatitis in rats after a single gene transfer *in vivo.*

### In vivo effect of asialo IFN-β in athymic nude mice model of HBV.

pHBV-HTD was complexed with asialofetuin-poly-L-lysine conjugate and cationic-liposome to make virus-like particles for liver-specific transfection (infectious liposome). The infectious liposome complex was injected in to the portal vein of nude mice via spleen. Seven days after in vivo transfection, mice were randomly selected and treated with intraperitoneal injections of physiological saline solution (PSS), IFN-β (10,000 IU/day) or asialo IFN-β (10,000 IU/day) for seven days.

Asialofetuin-poly-L-lysin conjugate and cationic liposome were prepared as described by Trubetskay et al. (Bioconjugate Chem. 3:323, 1992) and Karl et al. (Am. J. Med. Sci. 307:138, 1994). Since asialofetuin binds AGPR of hepatocytes and cationic liposome facilitates the fusion to cell membrane and delivery of DNA into hepatocytes, pHBV-HTD was complexed with asialofetuin-poly-L-lysine conjugate and cationic-liposome in order to accomplish liver-specific transfection. In brief, 50 µl of pHBV-HTD (400 µg/ml of DMEM), 100 µl of asialofetuin-poly-L-lysine conjugate (500 µg/ml of HBSS, pH7.4) and 100 µl of cationic-liposome (1,000 µg/ml of HBSS, pH7.7) were mixed in microcentrifuge tube. After 15 minute incubation at room temperature with mixing, the [asialofetuin-plu-L-lysine]-[DNA]-[cationic lipid] complex was filtered through 0.2 µm polycarbonate membrane filter (Poretics Corporation, CA) before transfection.

### Preparation of asialo IFN-β.

About 1 mg of human IFN-β (produced by human fibroblasts) was digested with 1 unit *Arthobacter ureafaciens*-derived neuraminidase (sialidase) in 1 ml of 5 mM formic acid (pH 3.5) at 37°C for 3 hrs (Figure 6). After hydrolysis, desialylated IFN-β was isolated on a C18 reversed-phase column (Zorbax PR-10, 4.6 x 300 mm; DuPont) with a linear gradient of acetonitrile in 0.1% trifluoroacetic acid.

### In vivo effect of asialo IFN-β in athymic nude mice model of HBV.

HBV particles were produced in all nude mice by *in vivo* transfection using infectious liposomes. In nude mice treated with PBS, HBV viremia continued to the end of the treatment (14 days after the transfection) (Figure 7). Sialylated natural IFN-β (10,000 IU/mouse/day for 7 days) did not demonstrate significant anti-viral effect. In contrast, asialo IFN-β (10,000 IU/mouse/day for 7 days) demonstrated substantial anti-viral effect as shown in Figure 7.

### Effect of human asialo IFN-β on HBV transfected human hepatoma cells

Asialoglycoprotein receptor bearing hepatoma cell lines were identified using [¹²¹I]-labeled asialo-orsomucoid (Schwartz et al., J. Biol. Chem. 256:8878, 1981). One asialoglycoprotein receptor expressing cell line HepG2 (American Type Culture Collection; Bethesda, MD: ATCC HB8065) was selected for transfection with HBV. HepG2 cells were transfected with pHBV-HTD as described below.

To examine the effect of asialo IFN-β and natural IFN-β on HBV-transfected HepG2 cells, 2x10⁵ transfected cells were treated with neither human natural IFN-β (1,000 IU/ml) or asialo IFN-β (1,000 IU/ml) or saline for 48 hours. Cytotoxicity was monitored using a colorimetric MTT cell proliferation assay as described by Mosmann (J. Immunol. Meth. 65:55, 1983).

### Asialo IFN-β is More Effective than the Natural IFN-β

Both asialo IFN-β and natural IFN-β were found to reduce HBV production by HBV transfected HepG2 cells. However, asialo IFN-β was found to be more effective than natural IFN-β. Asialo IFN-β reduced HBV production more than 5-fold, compared to control cells, while natural IFN-β reduced HBV production only 1.5 to 2-fold. Moreover, asialo IFN-β reduced HBsAg production by HBV transfected HepG2 cells 26-38%, while natural IFN-β reduced HBsAG production 33-40%.

The effect of asialo IFN-β and natural IFN-β on pHBV-HTD transfected SK-HEP cells was examined. These cells lack the asialoglycoprotein receptor. For these cells, asialo IFN-β was no more effective than natural IFN-β.

**Table 1**

| | HBsAg (ng/ml) | | |
|---|---|---|---|
| | 0 hr | 48 hr | 72 hr |
| Saline | 0.018±0.006 | 6.934±0.175 | 14.530±0.280 |
| IFN-β (1,000 IU/ml) | 0.029±0.003 | 2.747±0.090* | 3.830±0.266** |
| AS-IFN-β | | | |
| (1,000 IU/ml) | 0.042±0.008 | 2.618±0.093* | 3.830±0.266** |

| | | | |
|---|---|---|---|
| NOTE: Six hours after transfection, saline, IFN-β or asialo-IFN-β was added to culture medium (0 hr). Production of HBsAg from transfected HepG2 cells was significantly inhibited by IFN-β and AS-IFN-β by 48 hr treatment (*: P = 0.00004 vs. saline, **: P = 0.00004 vs. saline and P = 0.015 vs. IFN-β respectively by Student's *t* test). | | | |

The following methods were used in the experiments described in this section.

For these experiments, human asialo IFN-β was prepared from natural glycosylated IFN-β as described above. HBV virion production by HBV-transfected HepG2 cells was measured as described above. HBV surface antigen (HBsAg) was examined using a enzyme-linked immunosorbent assay (AUSZYME, Abbot Laboratories).

All cell lines were maintained in Eagle's MEM (M.A. Bioproducts; Walkersville, MD) supplemented with 10% fetal calf serum inactivated at 56°C for 30 min., 10 µM non-essential amino acids, 100 U/ml penicillin, and 100 µg/ml streptomycin. Cells used for *in vitro* testing were harvested from the monolayer cultures by treatment with 0.04% ethylene diaminetetraaetic (EDTA)/versine buffer in the absence of trypsin for 5 min. at 37°C.

Cells were transfected with pHBV-HTD (described above) by the calcium phosphate method (Mol. Biol. Cell. 7:2745, 1987) using 2 x 10⁵ cells and 3 µg of pHBV-HTD per 300 mm plate. After transfection, 30 µl of IFN-β (100 IU/µl) or asialo IFN-β (100 IU/µl) were applied every 6 hours for 48 hours to culture medium to a final concentration of 1,000 IU/ml. The same total volume of physiological saline was added to control cultures.

### Asialo-interferon

Asialo interferon used to treat hepatitis B and other conditions can be produced by removing a terminal sialic residue from interferon which is glycosylated and normally has such a residue by virtue of it having been produced in a eukaryotic cell. Thus, the interferon can be isolated from human fibroblasts which produce it naturally or from eukaryotic cells which have been manipulated so that they express a cloned interferon gene.

### Animal models

The methods described above may be used to prepare rodent models of other forms of hepatitis. Thus, other variant and mutant form of the hepatitis B virus may be used in place of adw2 variant used in the above-described experiments. Thus, the adw, adr(1), adr(2), ayr, ayw(1), ayw(2) or other variants may be used. In addition, the methods described above may also be used by those of skill in the art to prepare models of hepatitis C and hepatitis G.

### Treatment with Asialo-interferon

The method described above, and other techniques known to those skilled in the art, can be used to prepare asialo forms of glycosylated interferons. Thus, it may be possible to prepare asialo forms of interferon α₂ or other glycosylated human interferons. The glycosylated interferon can be used to treat a wide range of hepatic diseases or other diseases requiring administration of interferon to cells bearing the asialoglycoprotein receptor including: hepatitis B, hepatitis C, renal cell carcinoma, and hepatitis G.

The removal of a terminal sialic acid residue may be a useful method for modifying a wide range of other proteins produced in eukaryotic cells (naturally or by expression of a recombinant gene). This modification should produce an asialo-protein which can be more readily taken up by cells bearing the asialoglycoprotein receptor and is thus more effective.

### Use

The animal hepatitis models can be used for immunological and molecular studies of the pathologic process of hepatitis including studies of liver cell death. Importantly, the model can be used to screen potential therapeutics.

Mutational changes or deletions in the HBV genome have been identified and are believed to be associated with the development of severe forms of hepatitis; however, this hypothesis has not been tested *in vivo* because of the lack of an appropriate model system. The cellular functions of various HBV transactivator proteins and the possible involvement of these proteins in the cancer process have also not been examined in normal adult hepatocytes. This hypothesis may now be amenable to experimental evaluation using the animal hepatitis model described herein by preparing animals harboring variant or mutant or another virus. In addition, it is now possible to develop experimental models to test the anti-viral effect of therapeutic regimens *in vivo* and to investigate the pathogenicity of other hepatotrophic viruses, including hepatitis C virus.

Asialo-interferon β can be used to treat hepatitis B (or hepatitis C or hepatitis G) at dosages similar to or less than used by those skilled in the art for the natural form of human interferon. Because of the greater specificity, higher effective dosages will be possible with lower toxicity. Those skilled in the art will be able to determine the proper dosage through the use of animal models and dose escalation clinical trials. Of course, the effective dosage will generally be less than for natural interferon which has not been treated to remove a terminal sialic acid. Other forms of interferon can be treated similarly.

## Claims

1. Use of asialo-interferon for the preparation of a medicament for treating a patient infected with a hepatitis virus.

2. The use of claim 1 wherein the asialo-interferon is asialo-interferon-β.

3. The use of claim 1 or 2 wherein the hepatitis virus is hepatitis B virus.

4. The use of claim 1 or 2 wherein the hepatitis virus is hepatitis C virus.

5. The use of claim 1 or 2 wherein the hepatitis virus is hepatitis G virus.

6. The use of any one of claims 1 to 5 wherein the asialo-interferon is prepared from naturally-occurring human interferon.

7. The use of any one of claims 1 to 5 wherein the asialo-interferon is prepared from recombinant interferon.

8. The use of claim 6 or 7 wherein the asialo-interferon is obtainable by removing a terminal sialic acid from the interferon.

9. The use of claim 8 wherein the terminal sialic acid is an amino terminal sialic acid.

10. The use of claim 8 wherein the terminal sialic acid is removed by treating the interferon with sialidase.

## Patentansprüche

1. Verwendung von Asialo-interferon zur Herstellung eines Medikamentes zur Behandlung eines mit einem Hepatitis Virus infizierten Patienten.

2. Verwendung nach Anspruch 1, wobei das Asialo-interferon das Asialo-interferon-β ist.

3. Verwendung nach Anspruch 1 oder 2, wobei das Hepatitis Virus das Hepatitis-B-Virus ist.

4. Verwendung nach Anspruch 1 oder 2, wobei das Hepatitis Virus das Hepatitis-C-Virus ist.

5. Verwendung nach Anspruch 1 oder 2, wobei das Hepatitis Virus das Hepatitis-G-Virus ist.

6. Verwendung nach einem der Ansprüche 1 bis 5, wobei das Asialo-interferon aus natürlich vorkommende Human-interferon hergestellt wird,

7. Verwendung nach einem der Ansprüche 1 bis 5, wobei das Asialo-interferon aus rekombinantem Interferon hergestellt wird.

8. Verwendung nach Ansprüchen 6 oder 7, wobei das Asialo-interferon durch Entfernen einer terminalen Sialinsäure aus dem Interferon erhältlich ist.

9. Verwendung nach Anspruch 8, wobei die terminale Sialinsäure eine Aminoterminale-sialinsäure ist.

10. Verwendung nach Anspruch 8, wobei die terminale Sialinsäure durch Behandlung des Interferons mit Sialidase entfernt wird.

## Revendications

1. Utilisation d'asialo-interféron pour la préparation d'un médicament pour traiter un patient infecté par un virus de l'hépatite.

2. Utilisation selon la revendication 1 dans laquelle l'asialo-interféron est l'asialo-interféron-β.

3. Utilisation selon la revendication 1 ou 2 dans laquelle le virus de l'hépatite est un virus de l'hépatite B.

4. Utilisation selon la revendication 1 ou 2 dans laquelle le virus de l'hépatite est le virus de l'hépatite C.

5. Utilisation selon la revendication 1 ou 2 dans laquelle le virus de l'hépatite est le virus de l'hépatite G.

6. Utilisation selon l'une quelconque des revendications 1 à 5 dans laquelle l'asialo-interféron est préparé à partir d'interféron humain naturel.

7. Utilisation selon l'une quelconque des revendications 1 à 5 dans laquelle l'asialo-interféron est préparé à partir d'interféron recombinant.

8. Utilisation selon la revendication 6 ou 7 dans laquelle l'asialo-interféron peut être obtenu en éliminant l'acide sialique terminal de l'interféron.

9. Utilisation selon la revendication 8 dans laquelle l'acide sialique terminal est un acide sialique amino-terminal.

10. Utilisation selon la revendication 8 dans laquelle l'acide sialique terminal est éliminé par traitement de l'interféron avec de la sialidase.


### SEQUENCE LISTING

(1) GENERAL INFORMATION
   (i) APPLICANT: THE GENERAL HOSPITAL CORPORATION
   (ii) TITLE OF THE INVENTION: METHOD FOR TREATING HEPATITUS VIRUS INFECTION
   (iii) NUMBER OF SEQUENCES: 8
   (iv) CORRESPONDENCE ADDRESS:
      (A) ADDRESSEE: Fish & Richardson P.C.
      (B) STREET: 225 Franklin Street
      (C) CITY: Boston
      (D) STATE: MA
      (E) COUNTRY: USA
      (F) ZIP: 02110-2804
   (v) COMPUTER READABLE FORM:
      (A) MEDIUM TYPE: Diskette
      (B) COMPUTER: IBM Compatible
      (C) OPERATING SYSTEM: DOS
      (D) SOFTWARE: FastSEQ Version 2.0
   (vi) CURRENT APPLICATION DATA:
      (A) APPLICATION NUMBER: PCT/US96/----
      (B) FILING DATE: 27-SEP-1996
      (C) CLASSIFICATION:
   (vii) PRIOR APPLICATION DATA:
      (A) APPLICATION NUMBER: 60/004,357
      (B) FILING DATE: 27-SEP-1995
   (viii) ATTORNEY/AGENT INFORMATION:
      (A) NAME: Clark, Paul T
      (B) REGISTRATION NUMBER: 30,162
      (C) REFERENCE/DOCKET NUMBER: 00786/295WO1
   (ix) TELECOMMUNICATION INFORMATION:
      (A) TELEPHONE: 617-542-5070
      (B) TELEFAX: 617-542-8906
      (C) TELEX: 200154
(2) INFORMATION FOR SEQ ID NO:1:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (xi) SEQUENCE DESCRIPTION: SEQ ID NO:1:
      TGCGGGTCAC CATATTCTTG GGAACAAGA 29
(2) INFORMATION FOR SEQ ID NO:2:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (xi) SEQUENCE DESCRIPTION: SEQ ID NO:2:
      AGTCTAGACT CTGCGGTATT GTGAGGATTC TTG 33
(2) INFORMATION FOR SEQ ID NO:3:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (xi) SEQUENCE DESCRIPTION: SEQ ID NO:3:
      ATCTGGCACC ACACCTTCTA CAATGAGCTG CG 32
(2) INFORMATION FOR SEQ ID NO:4:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (xi) SEQUENCE DESCRIPTION: SEQ ID NO:4:
      CGTCATACTC CTGCTTGCTG ATCCACATCT GC 32
(2) INFORMATION FOR SEQ ID NO:5:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (xi) SEQUENCE DESCRIPTION: SEQ ID NO:5:
      GAGAATTCAA GCCTCCAAGC TGTGCCTGG 29
(2) INFORMATION FOR SEQ ID NO:6:
   (i) SEQUENCE CHARACTERISTICS:
      (A) LENGTH: 28 base pairs
      (B) TYPE: nucleic acid
      (C) STRANDEDNESS: single
      (D) TOPOLOGY: linear
   (xi) SEQUENCE DESCRIPTION: SEQ ID NO:6:
      GAAAGCTTCT GCGACGOGGC GATTGAGA 28
(2) INFORMATION FOR SEQ ID NO:7:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (xi) SEQUENCE DESCRIPTION: SEQ ID NO:7:
      ATCTGGCACC ACACCTTCTA CAATGAGCTG CG 32
(2) INFORMATION FOR SEQ ID NO:8:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (xi) SEQUENCE DESCRIPTION: SEQ ID NO:8:
      CGTCATACTC CTGCTTGCTG ATCCACATCT GC 32

### SEQUENCE LISTING

(1) GENERAL INFORMATION
   (i) APPLICANT
      (A) NAME: The General Hospital Corporation
      (B) STREET: 55 Fruit Street
      (C) CITY: Boston
      (D) STATE OR PROVINCE: MA
      (E) COUNTRY: USA
      (F) POSTAL CODE: 02114
   (ii) TITLE OF THE INVENTION: METHOD FOR TREATING VIRUS INFECTION
   (iii) NUMBER OF SEQUENCES: 8
   (iv) COMPUTER-READABLE FORM:
      (A) MEDIUM TYPE: Diskette
      (B) COMPUTER: IBM Compatible
      (C) OPERATING SYSTEM: Windows95
      (D) SOFTWARE: FastSEQ for Windows Version 2.0
   (v) CURRENT APPLICATION DATA:
      (A) APPLICATION NUMBER:
(2) INFORMATION FOR SEQ ID NO:1:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (xi) SEQUENCE DESCRIPTION: SEQ ID NO:1:
      TGCGGGTCAC CATATTCTTG GGAACAAGA 29
(2) INFORMATION FOR SEQ ID NO:2:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (xi) SEQUENCE DESCRIPTION: SEQ ID NO:2:
      AGTCTAGACT CTGCGGTATT GTGAGGATTC TTG 33
(2) INFORMATION FOR SEQ ID NO:3:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (xi) SEQUENCE DESCRIPTION: SEQ ID NO:3:
      ATCTGGCACC ACACCTTCTA CAATGAGCTG CG 32
(2) INFORMATION FOR SEQ ID NO:4:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (xi) SEQUENCE DESCRIPTION: SEQ ID NO:4:
      CGTCATACTC CTGCTTGCTG ATCCACATCT GC 32
(2) INFORMATION FOR SEQ ID NO:5:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (xi) SEQUENCE DESCRIPTION: SEQ ID NO:5:
      GAGAATTCAA GCCTCCAAGC TGTGCCTGG 29
(2) INFORMATION FOR SEQ ID NO:6:
   (i) SEQUENCE CHARACTERISTICS:
      (A) LENGTH: 28 base pairs
      (B) TYPE: nucleic acid
      (C) STRANDEDNESS: single
      (D) TOPOLOGY: linear
   (xi) SEQUENCE DESCRIPTION: SEQ ID NO:6:
      GAAAGCTTCT GCGACGCGGC GATTGAGA 28
(2) INFORMATION FOR SEQ ID NO:7:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (xi) SEQUENCE DESCRIPTION: SEQ ID NO:7:
      ATCTGGCACC ACACCTTCTA CAATGAGCTG CG 32
(2) INFORMATION FOR SEQ ID NO:8:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (xi) SEQUENCE DESCRIPTION: SEQ ID NO:8:
      CGTCATACTC CTGCTTGCTG ATCCACATCT GC 32